# EUROPEAN PATENT SPECIFICATION

(11) **EP 4 482 815 B1**
(45) Date of publication and mention of the grant of the patent: **15.04.2026**
(21) Application number: 23705573.6
(22) Date of filing: 21.02.2023
(51) Int. Cl.: C07C 11/02

(54) **PROCESS FOR THE OXIDATIVE DEHYDROGENATION OF ALKANES**
VERFAHREN FÜR DIE OXIDATIVE DEHYDRIERUNG VON ALKANEN
PROCÉDÉ DE DÉSHYDROGÉNATION OXYDATIVE D'ALCANES

(30) Priority: 22.02.2022 EP 22157964
(43) Date of publication of application: 01.01.2025
(73) Proprietor: SABIC Global Technologies B.V., 4612 PX Bergen op Zoom (NL)
(72) Inventor: BROEKHUIS, Robert, Bengaluru 562125 (IN); GAUTAM, Pankaj Singh, Bengaluru 562125 (IN); GU, Tian, Bengaluru 562125 (IN)
(74) Representative: Algemeen Octrooi- en Merkenbureau B.V.
(86) International application number: PCT/EP2023/054269
(87) International publication number: WO 2023/161209

(56) References cited:
- WO-A1-2014/090714
- CN-A- 103 965 002
- US-B2- 10 035 740

## Description

### FIELD

The present disclosure relates to a process for producing an alkene-containing product stream by oxidative dehydrogenation of the corresponding alkane. The present disclosure further relates to a reactor device for producing the alkene-containing product by the process.

### BACKGROUND

A known process for producing alkenes (olefins) such as ethylene and propylene is the steam cracking of different hydrocarbons. In particular, ethane, propane, naphtha, gas oils and other heavier molecules can be used as possible feedstock. A disadvantage of the steam-cracking process, especially in operations with heavier feedstock, is that additional by-products are produced which have to be separated off from the desired olefin product. Furthermore, steam cracking is an energy-intensive process that occurs at comparatively high temperatures in the range of about 800⁰C to 900⁰C. In this process, compounds with multiple unsaturated bonds are also always formed (e.g., acetylene, methyl acetylene, propadiene) which require elaborate separation, e.g., by extraction or selective hydrogenation.

A method that is known in principle as an alternative to the preparation of C2 - C4 olefins, in particular ethylene, is the oxidative dehydrogenation of the corresponding alkanes in the presence of a catalyst. In this process, the product is largely limited to alkenes with some amounts of carbon monoxide and carbon dioxide as by-products. The oxidative dehydrogenation (ODH) of ethane is thermodynamically favored and can be carried out at lower reaction temperatures without coke formation.

WO2010115108A1 describes a process for the oxidative dehydrogenation of ethane by contacting an ethane feed and an oxygen-containing gas in the presence of an oxidative dehydrogenation catalyst in an oxidative dehydrogenation reaction zone. This process oxidatively dehydrogenates at least a portion of the ethane. After the oxidative dehydrogenation of the alkanes in the product stream, unreacted oxygen is burned on a corresponding catalyst.

EP2716621A1 discloses a method and an apparatus for oxidative dehydrogenation of alkanes, comprising passing a feedstream comprising an alkane and oxygen by at least one catalyst contained in a fixed bed in a reactor device. This generates an alkene-containing product stream by oxidative dehydrogenation of the alkanes with oxygen in the presence of the catalyst, wherein the catalyst is diluted by using an inert material. EP2716622A1 discloses oxidative dehydrogenation of alkanes, wherein a diluent is introduced in the reactor device and the diluent is inert or comprises at least one inert component.

US10035740B2 describes a method of oxidatively dehydrogenating a hydrocarbon. It includes providing a first gaseous feedstream to a first adiabatic, catalytic reaction zone with less than a stoichiometric amount of oxygen and superheated steam. Then the hydrocarbon is oxidatively dehydrogenated in the first adiabatic, catalytic, reaction zone. Subsequently the effluent is cooled, additional oxygen added and the effluent stream reacted in at least one subsequent adiabatic reaction zone.

WO2014/090714 discloses an oxidative dehydrogenation process of alkanes to alkenes in a series of three reactors.

The article entitled "Ethylene production via Oxidative Dehydrogenation of Ethane using M1 catalyst" by Gaffney and Mason, Catalysis Today 285 (2017) 159-165, discloses the use of cooled multi-tubular reactors for a oxidative dehydrogenation process for producing ethane, as well as a partial oxidative dehydrogenation process for producing ethylene oxides. In such reactors, reaction heat is removed using heat transfer oil in the lower-temperature stages, and supercritical steam in the high-temperature stages.

A major concern in the ODH process is heat management, since significant heat is released by the exothermic reactions. ODH reactors are therefore prone to thermal runaway, i.e., the heat released as the oxidation reaction proceeds increases the temperature of the reaction mixture. This, in turn, not only increases the rate of the desired reaction (i.e., ODH to ethylene), but also of the undesired side reactions, such as partial or deep oxidation (i.e., oxidation to carbon monoxide or the total oxidation to carbon dioxide and water). An increased rate of these side reactions results in poorer selectivity to the desired ethylene product, which may negate any advantage over steam cracking, especially as the primary by-products of the side reactions (COx) do not provide added value, in contrast to the significant value for the hydrocarbon by-products from steam cracking.

Furthermore, the excess heat produced from the ODH reaction can exacerbate the side reactions and it would be easy for these self-accelerating side reactions to overpower the means of cooling, resulting in temperature hotspots in the reactor. Unless this is controlled, this would lead to damage to the catalyst, lower selectivity and poorer yields of the process and / or mechanical failures and safety concerns. Even with the use of heat transfer oils or other coolants in the high temperature stages, it is difficult to control the temperature and optimize the process, and avoiding thermal runaway requires expensive reactor designs. There has been mention of the use of thermal moderators such as inert diluents, moderator gases and selectivity modifiers such as superheated steam in various prior art sources. These seem to help in controlling the reaction temperature, such that high conversions and selectivities are achieved while suppressing the tendency towards thermal runaway. However, the introduction of such thermal moderator gases invariably renders such processes more energy intensive, which consequently may not be economically viable. Therefore, it is apparent that thermal runaway concerns have not been adequately addressed in an energy- and cost-efficient manner in the prior art.

It is, therefore, an object of some embodiments of the disclosure to provide a method for producing an alkene-containing product stream from an ODH reaction of alkane, wherein adverse effects of thermal runaway caused by a large amount of heat generation are prevented or minimized.

It is another object of some embodiments of the disclosure to provide for a method for producing an alkene-containing product stream from an ODH reaction of alkane, wherein the amount of unwanted products produced from either partial or deep oxidation is reduced.

It is yet another object of some embodiments of the disclosure to provide for a reactor device for producing an alkene-containing product stream by ODH of alkane that results in higher overall energy efficiency than the currently commercially available alkane steam crackers.

### SUMMARY

Illustrative embodiments of the present disclosure are directed to a process for producing an alkene-containing product stream by oxidative dehydrogenation. The process comprises (a) providing a primary feedstream comprising a C2 - C6 alkane through a reactor system having a total of "n" reaction stages in series, each of the reaction stages containing a catalyst bed, wherein "n" varies from 2 to 20; (b) providing a secondary feedstream comprising oxygen to each of the reaction stages, (c) producing an effluent stream comprising alkene, alkane, and oxygen from each of the reaction stages; (d) cooling the effluent stream from reaction stages 1 through "n-1" to generate an input feedstream that is fed to the immediate next reaction stage; and (e) producing the alkene-containing product stream in the "n" ^{th} reaction stage. In the process each of the reaction stages is substantially adiabatic. The primary feedstream comprises ≥ 75 % by volume of the C2 - C6 alkane and the combination of the primary feedstream, the secondary feedstream , and any additional streams provided to the reactor stages comprises >= 75 % by volume of C2 - C6 alkane. The effluent stream from each reaction stage comprises ≤ 0.5 mole % of oxygen.

In some embodiments, the primary feedstream or the input feedstream is combined with the secondary feedstream to constitute a combined feedstream at each reaction stage, such that the concentration of oxygen in the combined feedstream at each reaction stage ≤ 5.0 mole %.

In some embodiments, the effluent stream from each reaction stage comprises ≤ 0.3 mole % of oxygen.

In some embodiments, "n" varies from 3 to 10, preferably from 5 to 8.

In some embodiments, each reaction stage is at a pressure from 1 to 40 atmospheres, preferably from 10 to 25 atmospheres.

In some embodiments, the combined feedstream to each reaction stage is characterized by an inlet temperature (Tᵢₙ) and wherein Tᵢₙ is from 200⁰C to 450⁰C, preferably from 250⁰C to 350⁰C.

In some embodiments, the effluent stream from each reaction stage is characterized by an outlet temperature (Tₒᵤₜ) and wherein Tₒᵤₜ - Tᵢₙ ≥ 50⁰C at a given reaction stage.

In some embodiments, each of the reaction stages achieves an oxygen conversion of ≥ 80%.

In some embodiments, the catalyst bed comprises a Moₐ V_{b} Nb_{c} X_{d} catalyst, and X comprises at least one of the following: Te, Li, Sc, Na, Be, Mg, Ca, Sr, Ba, Ti, Zr, Hf, Y, Ta, Cr, Fe, Co, Ni, Ce, La, Zn, Cd, Hg, Al, Pb, As, Bi, Sb, U, Mn, and W.

In some embodiments, the combined feedstream to the first reaction stage comprises at least 75 % by volume of the C2 - C6 alkane.

The process according to any one or more of claims 1-9, wherein the C2 - C6 alkane is ethane and the alkene-containing product stream comprises ethylene.

The process according to any one or more of claims 1-10, wherein the primary feedstream comprises at least 90 % by volume of ethane.

In some embodiments, the overall conversion of ethane of all the reaction stages is from 10% to 50%, preferably from 20% to 40%.

In some embodiments, the overall selectivity of ethylene at all the reaction stages is ≥ 90%.

Various embodiments of the present disclosure are also directed to a reactor apparatus for producing an alkene-containing product stream by oxidative dehydrogenation, according to any the processes described herein.

### BRIEF DESCRIPTION OF THE DRAWINGS

The above and other features, aspects and advantages of the present disclosure are better understood when the following detailed description of the disclosure is read with reference to the accompanying drawings, in which:
Figure 1 is a reactor system in accordance with one embodiment of the present disclosure;
Figure 2 is a reactor system with six reaction stages in accordance with one embodiment of the present disclosure;
Figure 3 shows a plot of process gas temperature, ethane conversion, and ethylene selectivity versus number of reaction stages in accordance with one embodiment of the present disclosure; and
Figure 4 shows a plot of total catalyst volume and inlet temperature at a first reaction stage versus the number of reaction stages in accordance with one embodiment of the present disclosure.

### DETAILED DESCRIPTION

The present inventors have found that the above stated goals can be achieved by producing an alkene-containing product stream by ODH of corresponding alkane, through a reactor system comprising multiple reaction stages, with a secondary feed of oxygen at each reaction stage and inter-stage cooling, in which each of the reaction stages is substantially adiabatic. The controlling of the temperature is accomplished, at least partially, by limiting the amount of oxygen at each stage, thus limiting the amount of reaction that occurs. Furthermore, the inventors have found that it is possible to advantageously conduct an ODH process at the process conditions described herein, in which there is no need for any substance to be added to the reactor feed as a thermal moderator or a selectivity modifier. This allows for a more energy-efficient operation. For convenience, as used herein, the term "reaction stage" comprises a catalytic bed in the reactor system for catalytically producing an alkene containing product stream from the corresponding alkane primary feedstream through an ODH process.

Operation under substantially adiabatic conditions enables the reaction mixture inside the reaction stage to absorb (most of) the heat of the reaction and emerge at the outlet of the reaction stage at a higher temperature than that at the inlet. Furthermore, controlling the amount of oxygen in substantially adiabatic conditions allows this limiting reactant to be substantially or fully converted in the reaction stage. Therefore, when a sufficiently low temperature of the feed is established at the inlet of each reaction stage, the resulting outlet temperature is predictable and within a suitable range, thereby establishing passive thermal control and avoiding damaging temperatures anywhere in the reactor system. This helps to, first, prevent the formation of an excessive amount of partial or deep oxidation products (i.e., improves selectivity), and second, prevent catalyst or equipment damage due to thermal runaway.

Accordingly, the present disclosure relates to a process for producing an alkene-containing product stream by oxidative dehydrogenation comprising the steps of,
a) providing a primary feedstream comprising a C2 - C6 alkane through a reactor system having a total of "n" reaction stages in series, each of the reaction stages containing a catalyst bed, wherein "n" varies from 2 to 20;
b) providing a secondary feedstream comprising oxygen to each of the reaction stages;
c) producing an effluent stream comprising alkene, alkane, and oxygen from each of the reaction stages;
d) cooling the effluent stream from reaction stages 1 through "n-1" to generate an input feedstream that is fed to the immediate next reaction stage; and
e) producing the alkene-containing product stream in the "n" ^{th} reaction stage;
wherein each of the reaction stages is substantially adiabatic and wherein the primary feedstream comprises ≥ 75 % by volume of the C2 - C6 alkane and the combination of the primary feedstream, the secondary feedstream, and any additional streams provided to the reactor stages comprises >= 75 % by volume of C2 - C6 alkane. The effluent stream from each reaction stage comprises <= 0.5 mole % of oxygen.

By application of the disclosure, the foregoing objects are met, at least in part. The same will now be described in more details.

A method of carrying out ODH of a C2 - C6 alkane for producing an alkene-containing product stream is carried out by the following steps:
Step a: providing a primary feedstream comprising a C2 - C6 alkane that passes through a reactor system having "n" total reaction stages. All the stages are preferably in series and each of the reaction stages contains a catalyst bed. In accordance with the various embodiments of the disclosure, "n" preferentially varies from 2 to 20. The primary feedstream comprising the alkane is first fed into the first reaction stage of the reactor system.

The catalyst used in the present disclosure may have the following composition:

Moₐ V_{b} Nb_{c}X_{d};

wherein X = at least one of the following:
Te, Li, Sc, Na, Be, Mg, Ca, Sr, Ba, Ti, Zr, Hf, Y, Ta, Cr, Fe, Co, Ni, Ce, La, Zn, Cd, Hg, Al, Pb, As, Bi, Sb, U, Mn, and W; and
a = 0.05 to 0.9
b = 0.1 to 0.4
c = 0.001 to 0.2
d = 0.001 to 1.0

The values of a, b, c and d constitute relative gram-atoms of the elements Mo, V, Nb, and X, respectively. The elements are present in combination with oxygen in the form of various oxides. The catalyst bed may comprise a supported catalyst. Suitable supports include silica, aluminum oxide, silicon carbide, zirconia, titania, and mixtures thereof. In principle any catalyst with sufficient activity and selectivity properties which is stable under the given reaction conditions can be used. The catalyst may take the form of a bulk particulate catalyst, a supported particulate catalyst, a supported structured catalyst (for example, a catalytic foam or a monolith), or any other form that promotes the reaction of the gaseous species over the solid catalyst.

Step b: providing a secondary feedstream comprising oxygen to each of the reaction stages. The temperature at which the secondary feedstream comprising oxygen is combined with the primary feedstream must be less than the temperature for which autoignition can occur. The "autoignition temperature" of a gas mixture, as defined herein, is the temperature at which the gas will ignite and sustain the combustion in the absence of an external ignition source. Therefore, in order to avoid autoignition, preferably, the temperature at which the secondary feedstream comprising oxygen is combined with the primary feedstream is < 350⁰C. Pure oxygen, oxygen-enriched air or air may be used for the process, preferably oxygen having a purity of at least 90.0% preferably at least 99.0%, most preferably at least 99.5%.

Step c: producing an effluent stream comprising alkene, alkane and oxygen from each of the reaction stages. The effluent stream may also contain other species such as unreacted gases which were present in the primary feedstream and/or the species produced by the reaction viz. CO, CO₂, and oxygenated organic species such as acetic acid.

Step d: cooling the effluent stream from reaction stages 1 through "n-1" to generate an input feedstream that is fed to the next reaction stage. The cooling is carried out via an interstage cooling unit which can be a heat exchanger or any other well-known equipment for cooling gaseous streams. It is known in the art that the reaction temperature can be controlled by directly cooling the reaction zone(s) and that for this it is necessary to select a coolant temperature in the vicinity of the reaction process temperature. The process according to the present disclosure does not pose any restrictions on the coolant temperature. Therefore, cooling may, for example, be conducted by steam-raising instead of using expensive cooling solutions such as those using molten salts or supercritical fluids.

The interstage cooling unit cools the effluent stream, to generate the input feedstream that is fed to the next reaction stage. According to the disclosure, the primary feedstream of the first stage or the input feedstreams of the subsequent stages combines with the secondary feedstream of oxygen to each reaction stage to constitute a combined feedstream for that particular reaction stage.

In order to operate such a reaction process, consideration must be given to certain safety factors in order to avoid combustion or explosion of the feed mixture. An important factor is the limiting oxygen concentration (LOC), which is defined as the lowest oxygen mole concentration in a mixture of oxygen with other gases comprising at least one flammable substance that supports combustion when a source of ignition is provided. The LOC depends on process conditions such as the composition of the other components of the gas mixture, as well as the pressure and temperature of the process. The LOC, therefore, represents an upper bound to the oxygen concentration in the ODH reactor feed. Prior art processes often select the highest oxygen concentration that they feel is safely below this upper bound, as this may result in higher rates of reaction or greater per-pass conversions. For example, in the ODH of ethane, the LOC may be around 10 mol%, due to which practitioners of prior art may select an oxygen concentration in the reactor feed of ~ 8 - 9 mol%.

In the present disclosure, the inventors have determined that the concentration of oxygen in the combined feedstream at each reaction stage can be advantageously selected at a value well below the LOC. Accordingly, the concentration of oxygen in the combined feedstream at each reaction stage is ≤ 5.0 mole percent, preferably from 1.0 to 5.0 mole percent, more preferably from 2.0 to 4.0 mole percent. The effluent stream produced by each reaction stage comprises ≤ 0.5 mole percent oxygen, preferably ≤ 0.3 mole percent oxygen.

By controlling the concentration of oxygen, and providing sufficient catalyst for the reaction to proceed, each reaction stage achieves an oxygen conversion of ≥ 80%, preferably ≥ 90%. Therefore, the oxygen fed to each reaction stage is nearly or fully consumed so that the effluent stream coming out of that reaction stage is at a higher temperature, before it enters the interstage cooling unit. This temperature is termed as an outlet temperature (Tₒᵤₜ). Cooling reduces the temperature of the effluent stream to a lower temperature, so that the secondary feed of oxygen can be introduced safely in the next reaction stage. The combined feedstream to each reaction stage is characterized by an inlet temperature (Tᵢₙ). In the present disclosure, Tᵢₙ varies from 200⁰C to 450⁰C, preferably from 250⁰C to 350⁰C. The inlet temperature is always lower than the outlet temperature. When the process is carried out under substantially adiabatic conditions and the reaction selectivities are known under the conditions traversed, this allows one to infer the outlet temperature by heat balance. Thereby, the temperature rise across each reaction stage is passively controlled and thermal runaway is prevented. In the present disclosure, Tₒᵤₜ - Tᵢₙ ≥ 50⁰C may be achieved at any given reaction stage. This indicates that the temperature rise across a particular reaction stage may be ≥ 50⁰C or ≥ 100⁰C. The permissible temperature rise across a particular reaction stage is primarily controlled by the need to prevent thermal runaway and secondarily by the need to retain good selectivity (and suppress the side reactions). Each reaction stage may be maintained at a pressure from 1 to 40 atmospheres, preferably from 10 to 25 atmospheres.

Step e: producing the alkene-containing product stream in the "n" ^{th} reaction stage. The "n" ^{th} reaction stage referred to herein is the "last reaction stage" from which the final alkene-containing product stream is obtained in accordance with the various embodiments of the disclosure.

The total number of reaction stages is determined by practical considerations. The total amount of oxygen provided for reacting with the primary feedstream is split between the total number of reaction stages. With more reaction stages, less oxygen is available to be converted in each reaction stage, generating a smaller amount of reaction heat, and a correspondingly lower temperature rise. If we desire to maintain the outlet temperature of a reaction stage below a certain limit (for example, to maintain a suitable selectivity), having more reaction stages allows each reaction stage to be operated with higher inlet temperatures. Higher temperatures generally give rise to higher average reaction rates and hence smaller reactor apparatus sizes or catalyst volume required. On the other hand, more reaction stages demand more interstage heat exchangers and more complex control strategies, which may imply higher costs with a large number of reaction stages. Therefore, determining a suitable number of reactors is an important aspect to the disclosure. Preferably, "n", varies from 3 to 10, more preferably from 5 to 8.

In various embodiments of the present disclosure, each of the reaction stages is substantially adiabatic. By the use of the particular configuration of the reactor system, adiabatic or near-adiabatic ODH of alkanes can be achieved. While adiabatic conditions are desired, in practice only near-adiabatic conditions can be maintained. This is true even though reference is commonly made to the use of adiabatic reactors. Those skilled in the art will recognize that there is some heat loss from such adiabatic reactors so they are not perfectly adiabatic. Accordingly, the use of the term "substantially adiabatic" throughout this disclosure may therefore refer to such near-adiabatic conditions, which can be defined as from 10% or less heat transfer or heat loss from the reactor (relative to the total heat generated).

The C2 - C6 alkane content in the primary feedstream is ≥ 75% by volume, more preferably ≥ 90 % by volume. Furthermore, the feed stream obtained from combining all feeds to all reaction stages, i.e., the combination of the primary feedstream, the secondary feedstream, and any additional streams provided to the reactor stages, comprises at least 75% by volume of C2 - C6 alkane, more preferably at least 90% by volume of C2 - C6 alkane. According to the present disclosure, it is not required to add any steam or inert compounds to the reactor system, for example to serve as a thermal moderator or a selectivity modifier, though it would not outside the scope of the disclosure to add these

The present disclosure can generally be applied to any C2 - C6 alkanes. This means that the alkanes of the present disclosure contains 2 to 6, preferably 2 to 5, most preferably 2 or 4 carbon atoms. The alkane can be linear or branched. Examples of preferred alkanes are, but not limited to, ethane, propane, n-butane, isobutane, pentane and hexane. The primary feedstream may comprise more than one C2 - C6 alkane. Preferably, the alkane is ethane and the alkene-containing product stream comprises ethylene. In this case, the primary feedstream may be a commercial grade of ethane. It is known that commercial grades of ethane may contain methane, propane, and trace quantities of hydrogen sulfide, CO₂, and nitrogen. A series arrangement of the reaction stages provides a cumulative increase in the conversion of ethane. The unreacted ethane from any stage can be further converted in subsequent stages. The oxygen for the conversion is supplied as the secondary feedstream of each reactor. Thus, the series arrangement of the reaction stages enables the safe achievement of the conversion of ethane for all the reaction stages.

In accordance with the various embodiments of the disclosure, a particularly advantageous process is obtained when the overall conversion of ethane of all the reaction stages is from 10% to 50%, preferably from 20% to 40%. The overall ethylene selectivity resulting from all the reaction stages is ≥ 90%, preferably ≥ 95%. Assuming that the oxygen is equally distributed among all the reaction stages and that an ethylene selectivity of 97% is maintained, the total number of reaction stages required to achieve an overall 30% ethane conversion can be directly correlated to the concentration of oxygen in the combined feedstream in the first reaction stage. This is demonstrated in Table 1. Note that the disclosure does not require that all stages have exactly the same amount of oxygen fed to them.

**Table 1: Total number of reaction stages vs. concentration of oxygen in the combined feedstream in the first reaction stage to achieve 30% overall ethane conversion at 97% ethylene selectivity**

| Total Number of reaction stages required | Concentration of oxygen in the combined feed stream to the first reaction stage (mol %) |
|---|---|
| 2 | 8.1 |
| 3 | 5.6 |
| 4 | 4.2 |
| 5 | 3.4 |
| 6 | 2.9 |
| 7 | 2.5 |
| 8 | 2.2 |

According to the disclosure, converting a certain amount of ethane requires a definite amount of oxygen consumption. The actual amount of oxygen consumption depends on the ethylene selectivity. The higher the oxygen feed concentration in each reaction stage, the fewer reaction stages are required to achieve the ethane conversion, as shown in Table 1. Furthermore, the temperature rise across any reaction stage can be directly correlated to the concentration of oxygen in the combined feedstream. From this, the maximum inlet temperature at each reaction stage can be determined to prevent exceeding the chosen temperature limit.

The disclosure further relates to a reactor apparatus for producing an alkene containing product stream by ODH of a C2 - C6 alkane. The reactor apparatus in the present disclosure is used for producing an alkene-containing product stream by ODH comprising the steps of,
a) providing a primary feedstream comprising a C2 - C6 alkane through a reactor system having a total of "n" reaction stages in series, each of the reaction stages containing a catalyst bed, wherein "n" varies from 2 to 20,
b) providing a secondary feedstream comprising oxygen to each of the reaction stages,
c) producing an effluent stream comprising alkene, alkane, and oxygen from each of the reaction stages;
d) cooling the effluent stream from reaction stages 1 through "n-1" to generate an input feedstream that is fed to the immediate next reaction stage; and
e) producing the alkene-containing product stream in the "n" ^{th} reaction stage;
wherein each of the reaction stages is substantially adiabatic and wherein the primary feedstream comprises ≥ 75 % by volume of the C2 - C6 alkane and the combination of the primary feedstream, the secondary feedstream, and any additional streams provided to the reactor stages comprises >= 75 % by volume of C2 - C6 alkane. The effluent stream from each reaction stage comprises <= 0.5 mole % of oxygen.

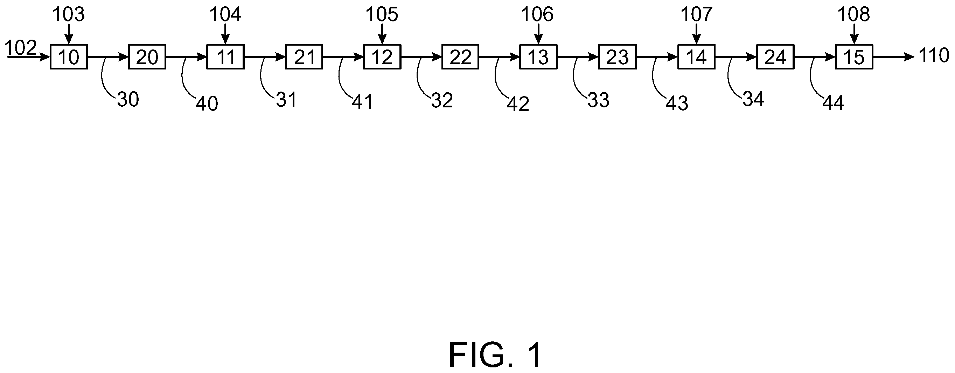

It has now been determined that a commercially viable process for the ODH of alkane to alkene may suitably comprise more than one reaction stage. The reaction stages may be in separate reactor vessels or they can be combined into a single common reactor vessel. Accordingly, in one embodiment, the reactor system may comprise a series of reactor vessels, wherein each reactor vessel comprises a single reaction stage containing a catalyst bed. A schematic representation of the reactor system and some of the method steps according to such an embodiment is shown in Figure 1.

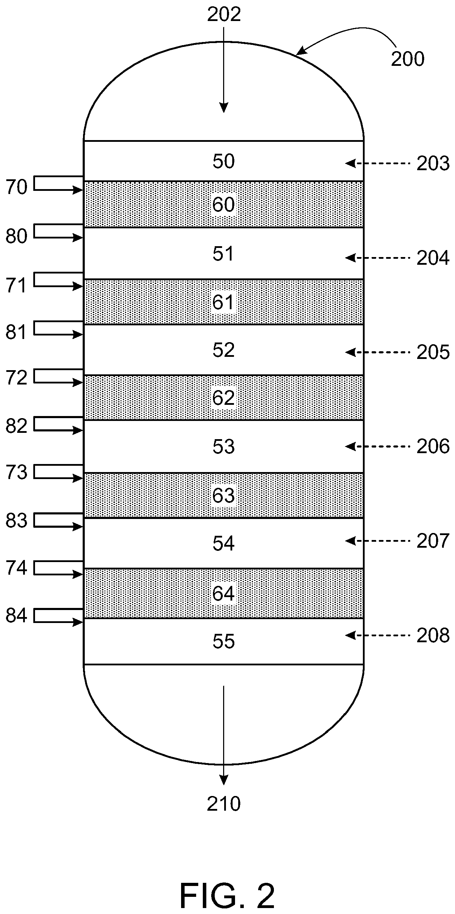

In another embodiment, the reactor system can comprise multiple reaction stages in series enclosed within a single vessel. Each of such reaction stages contains a catalyst bed. A schematic representation of the reactor system and some of the method steps according to such an embodiment is shown in Figure 2. It is noted that the present disclosure is not limited to the configuration of Figure 1 and / or Figure 2.

To put the present method in context, a process for producing an alkene-containing product stream by ODH will now be described.

Figure 1 shows a reactor system comprising six reaction stages in series from 10 to 15. Each of stages 10 to 15 is a separate fixed bed reactor containing a catalyst bed. The primary feedstream comprising ethane 102 enters reaction stage 10. A secondary feedstream comprising oxygen 103 is fed to reaction stage 10. Primary feedstream 102 and secondary feedstream 103 together constitute the combined feedstream for reaction stage 10. The combined feedstream is characterized by its temperature (Tᵢₙ). Effluent stream 30 is produced from reaction stage 10 comprising ethylene, unreacted ethane and unreacted oxygen. This is characterized by the outlet temperature (Tₒᵤₜ). It is noted that, according to the disclosure, at any given reaction stage, Tₒᵤₜ - Tᵢₙ ≥ 50⁰C. Effluent stream 30 enters the interstage cooling unit 20 wherein it is further cooled. Cooling unit 20 may be a shell-and-tube heat exchanger or other heat exchange equipment known in the art. Cooling unit 20 generates input feedstream 40 which is fed to the next reaction stage 11. Input feedstream 40 and secondary feedstream 104 together constitute the combined feedstream for 11.

Secondary feedstreams of oxygen in Figure 1, for reaction stages 10 to 15, are marked from 103 to 108 successively. The molar concentration of oxygen in each of feedstreams 103 to 108 may be the same or different. The flowrates of secondary feedstreams 103 to 108 may be the same or different. Effluent streams are marked from 30 to 34 for reaction stages 10 to 14 successively according to Figure 1. Interstage cooling units 21 to 24 are similar to cooling unit 20 and can be heat exchangers or any other well-known equipment for cooling, as described before. Input feedstreams marked as 40 to 44 are fed to reaction stages 11 to 15 successively. The process repeats for each of the reaction stages till reaction stage 15, which finally produces ethylene-containing product stream 110. In Figure 1, six reaction stages have been depicted, but it is understood that the disclosure is not limited to six reaction stages only.

Figure 2 depicts six reaction stages 50 to 55 in series each containing a catalyst bed, all six reactors enclosed within a single reactor apparatus 200. The primary feedstream comprising ethane 202 enters reaction stage 50. A secondary feedstream comprising oxygen 203 is fed to reaction stage 50. Primary feedstream 202 and secondary feedstream 203 together constitute the combined feedstream for reaction stage 50. The combined feedstream is characterized by the inlet temperature (Tᵢₙ). Effluent stream 70 is produced from reaction stage 50 comprising ethylene, unreacted ethane and unreacted oxygen. This is characterized by the outlet temperature (Tₒᵤₜ). It is noted that, according to the disclosure, at any given reaction stage, Tₒᵤₜ - Tᵢₙ ≥ 50⁰C. Effluent stream 70 enters the interstage cooling unit 60 wherein it is further cooled. For example, cooling may be effected by inserting cooling coils in the space between two adjacent catalyst beds. Alternatively, the effluent from the reaction apparatus may be extracted and passed through a heat exchanger, for example, a shell and tube type exchanger, before being redirected to the reaction apparatus to pass through the next reaction stage in sequence. Interstage cooling unit 60 generates input feedstream 80 which is fed to the next reaction stage 51. Input feedstream 80 and secondary feedstream 204 together constitutes the combined feedstream for reaction stage 51.

Secondary feedstreams of oxygen in Figure 2, for reaction stages 50 to 55, are marked from 203 to 208 successively. The molar concentration of oxygen in each of secondary feedstreams 203 to 208, may be same or different. The flowrates of secondary feedstreams 203 to 208 may be the same or different. Effluent streams are marked from 70 to 74 for reaction stages 50 to 54 successively according to Figure 2. Interstage cooling units 61 to 64 are similar to cooling unit 60 and can be heat exchangers or any other well-known equipment for cooling. Input feedstreams marked from 80 to 84 are fed to the reaction stages 51 to 55 successively, in Figure 2. The process repeats for each of the reaction stages till reaction stage 55, which finally produces ethylene-containing product stream 210. In Figure 2, six reaction stages have been depicted, but it is understood that the invention is not limited to six reaction stages only.

The present disclosure will now be described using the following non-limiting examples to demonstrate the practice and advantages thereof. It is understood that the examples are given by way of illustration and are not intended to limit the specification of the claims to follow in any manner.

### MATHEMATICAL MODEL:

The following examples draw on published information in the open literature, which is commonly used in the adiabatic reactor configurations examined by reactor modeling and simulation. To describe the reaction kinetics of the ODH chemistry, a power-law kinetic model was fit to publicly available experimental results for conversions and selectivities over a catalyst comprising Mo, V, Nb and Te. The model is characterized by positive, below-unity reaction orders in both ethane and oxygen partial pressure, for both the desired reaction to ethylene and the side reaction to CO and / or CO₂. The latter has the higher activation energy, consistent with deteriorating selectivity towards ethylene as temperature increases. A mathematical model of a conventional, adiabatic, packed-bed reactor was constructed.

### EXAMPLE 1:

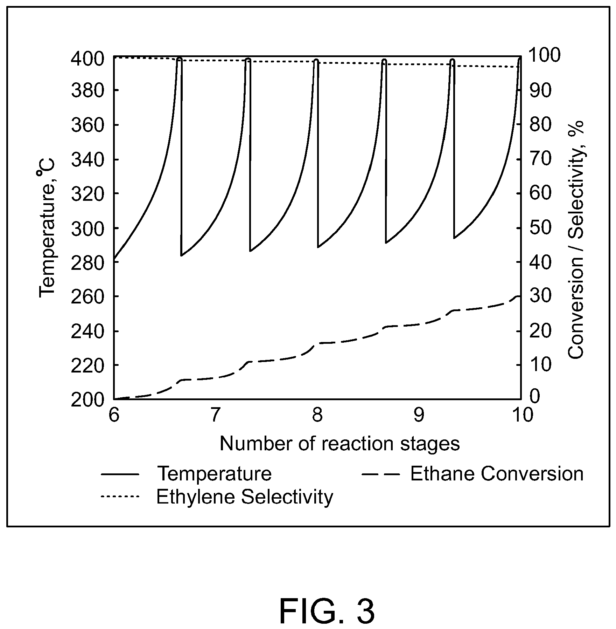

The mathematical model described above was applied to a case of six reaction stages in a series configuration, the first in sequence receiving an ODH primary feed containing 2.8 × 10⁴ kmol/hr ethane. An oxygen feed of 4.9 × 10³ kmol/hr is split into six equal portions, added to the feed of each of the six reaction stages. These feed conditions are selected to accomplish the production of 1800 kta ethylene. The temperature of the gaseous feed to the first reaction stage is at 283⁰C, and the pressure at 20 bar (absolute). In the subsequent reaction stages, the inlet temperature (Tᵢₙ) is increased by 3⁰C at each reaction stage (i.e. the inlet temperature is 286⁰C in the second reaction stage, and 289⁰C in the third reaction stage and so on), which results in an outlet temperature from each stage of about 400⁰C. The calculated profiles of process gas temperature, ethane conversion, and selectivity towards ethylene are shown in Figure 3. Each reaction stage achieves nearly full conversion of the oxygen in its feed. For the given flow rate, a total of 611 m³ of catalyst volume is used to achieve 30 % conversion of ethane. The overall ethylene selectivity achieved is ~ 97%. As a result of changing input feedstream concentrations, each reaction stage will require different catalyst volume to fully convert the oxygen. In the case modeled, the six reaction stages have catalyst volumes of 38 m³, 83 m³, 104 m³, 119 m³, 130 m³, and 137 m³ in order from first to sixth stage.

### EXAMPLE 2:

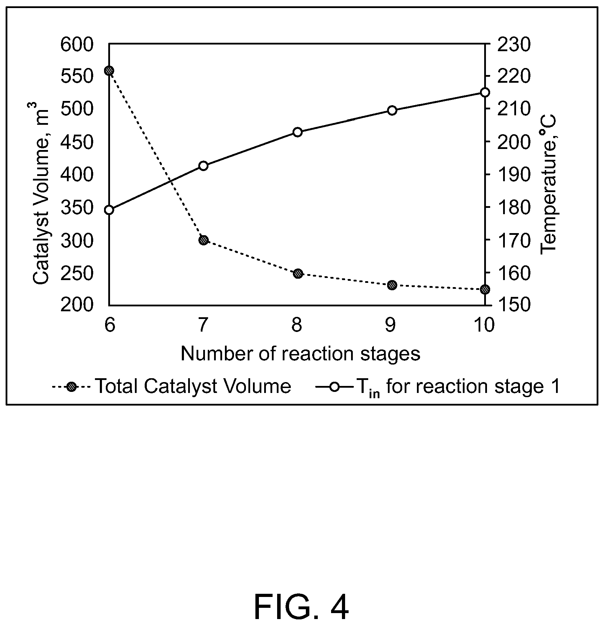

The same mathematical model was used for a case with the same ODH feed composition and flow rate as in Example 1, but with different numbers of reaction stages. The inlet temperature is set as in Example 1, and the outlet temperature at each reaction stage has been similarly constrained to be around 400⁰C, and the corresponding catalyst volumes were calculated. Figure 4 shows the relation between the total catalyst volume and the number of reaction stages. It also shows the relation between the inlet temperature to the first reaction stage and the number of reaction stages. We can see that the total catalyst volume decreases sharply from four to six reaction stages, after which the benefit of adding more reaction stages diminishes.

While embodiments of the disclosure have been shown and described, modifications thereof can be made without departing from the spirit and teachings of the disclosure. The embodiments and examples described herein are exemplary only, and are not intended to be limiting. Many variations and modifications of the invention disclosed herein are possible and are within the scope of the disclosure. Accordingly, the scope of protection is not limited by the description set out above but is only limited by the claims which follow. Each and every claim is incorporated into the specification as an embodiment of the present disclosure. Thus, the claims are a further description and are an addition to the detailed description of the present disclosure.

## Claims

1. A process for producing an alkene-containing product stream by oxidative dehydrogenation, comprising:
a) providing a primary feedstream comprising a C2 - C6 alkane through a reactor system having a total of "n" reaction stages in series, each of the reaction stages containing a catalyst bed, wherein "n" varies from 2 to 20,
b) providing a secondary feedstream comprising oxygen to each of the reaction stages,
c) producing an effluent stream comprising alkene, alkane, and oxygen from each of the reaction stages;
d) cooling the effluent stream from reaction stages 1 through "n-1" to generate an input feedstream that is fed to the immediate next reaction stage; and
e) producing the alkene-containing product stream in the "n" ^{th} reaction stage;
wherein;
each of the reaction stages is substantially adiabatic; the primary feedstream comprises >= 75 % by volume of the C2-C6 alkane and
the combination of the primary feedstream, the secondary feedstream, and any additional streams provided to the reactor stages comprises ≥ 75 % by volume of the C2 - C6 alkane; and
the effluent stream from each reaction stage comprises ≤ 0.5 mole % of oxygen.

2. The process according to claim 1, wherein the primary feedstream or the input feedstream is combined with the secondary feedstream to constitute a combined feedstream at each reaction stage, such that the concentration of oxygen in the combined feedstream at each reaction stage ≤ 5.0 mole %.

3. The process according to any one or more of claims 1-2, wherein the effluent stream from each reaction stage comprises ≤ 0.3 mole % of oxygen.

4. The process according to any one or more of claim 1-3, wherein "n" varies from 3 to 10, preferably from 5 to 8.

5. The process according to any one or more of claim 1-4, wherein each reaction stage is at a pressure from 1 to 40 atmospheres, preferably from 10 to 25 atmospheres.

6. The process according to any one or more of claim 1-5, wherein the combined feedstream to each reaction stage is **characterized by** an inlet temperature (Tᵢₙ) and wherein Tᵢₙ is from 200°C to 450⁰C, preferably from 250°C to 350°C.

7. The process according to any one or more of claim 1-6, wherein the effluent stream from each reaction stage is **characterized by** an outlet temperature (Tₒᵤₜ) and wherein Tₒᵤₜ - Tᵢₙ ≥ 50⁰C at a given reaction stage.

8. The process according to any one or more of claims 1-7, wherein each of the reaction stages achieves an oxygen conversion of ≥ 80%.

9. The process according to any one or more of claims 1-8, wherein the catalyst bed comprises a Moₐ V_{b} Nb_{c} X_{d} catalyst, wherein X = at least one of the following: Te, Li, Sc, Na, Be, Mg, Ca, Sr, Ba, Ti, Zr, Hf, Y, Ta, Cr, Fe, Co, Ni, Ce, La, Zn, Cd, Hg, Al, Pb, As, Bi, Sb, U, Mn, and W.

10. The process according to any one or more of claims 1-9, wherein the combination of the primary feedstream, the secondary feedstream, and any additional streams provided to the reactor stages comprises at least 90 % by volume of the C2 - C6 alkane.

11. The process according to any one or more of claims 1-9, wherein the C2 - C6 alkane is ethane and the alkene-containing product stream comprises ethylene.

12. The process according to any one or more of claims 1-10, wherein the primary feedstream comprises at least 90 % by volume of ethane.

13. The process according to any one or more of claims 1-12, wherein the overall conversion of ethane of all the reaction stages is from 10% to 50%, preferably from 20% to 40%.

14. The process according to any one or more of claims 1-13, wherein the overall selectivity of ethylene at all the reaction stages is ≥ 90%.

15. The process according to any one or more of claims 1-14, wherein reactor system is free of adding any steam or inert compounds to the reactor system.

## Patentansprüche

1. Prozess zur Herstellung eines alkenhaltigen Produktstroms durch oxidative Dehydrierung, umfassend:
a) Bereitstellen eines primären Zufuhrstroms, der ein C2 - C6 Alkan umfasst, durch ein Reaktorsystem, das insgesamt "n" Reaktionsstufen in Reihe aufweist, wobei jede der Reaktionsstufen ein Katalysatorbett enthält, wobei "n" von 2 bis 20 variiert,
b) Bereitstellen eines sekundären Zufuhrstroms, der Sauerstoff umfasst, für jede der Reaktionsstufen,
c) Herstellen eines Abflussstroms, der Alken, Alkan und Sauerstoff aus jeder der Reaktionsstufen umfasst;
d) Kühlen des Abflussstroms aus Reaktionsstufen 1 bis "n-1", um einen Eingangszufuhrstrom zu erzeugen, welcher der unmittelbar nächsten Reaktionsstufe zugeführt wird; und
e) Herstellen des alkenhaltigen Produktstroms in der "n" ^{-ten} Reaktionsstufe;
wobei;
jede der Reaktionsstufen im Wesentlichen adiabatisch ist; der primäre Zufuhrstrom >= 75 Vol.-% des C2-C6-Alkans umfasst und
die Kombination aus primärem Zufuhrstrom, sekundärem Zufuhrstrom und beliebigen zusätzlichen den Reaktorstufen bereitgestellten Strömen ≥ 75 Volumen-% des C2 - C6 Alkans umfasst; und
der Abflussstrom jeder Reaktionsstufe ≤ 0,5 Mol-% an Sauerstoff umfasst.

2. Prozess nach Anspruch 1, wobei der primäre Zufuhrstrom oder der Eingangszufuhrstrom mit dem sekundären Zufuhrstrom kombiniert wird, um einen kombinierten Zufuhrstrom in jeder Reaktionsstufe darzustellen, sodass die Sauerstoffkonzentration im kombinierten Zufuhrstrom in jeder Reaktionsstufe ≤ 5,0 Mol-% ist.

3. Prozess nach einem oder mehreren der Ansprüche 1-2, wobei der Abflussstrom aus jeder Reaktionsstufe ≤ 0,3 Mol-% an Sauerstoff umfasst.

4. Prozess nach einem oder mehreren der Ansprüche 1-3, wobei "n" zwischen 3 und 10, vorzugsweise zwischen 5 und 8 variiert.

5. Prozess nach einem oder mehreren der Ansprüche 1-4, wobei jede Reaktionsstufe auf einem Druck von 1 bis 40 Atmosphären, vorzugsweise von 10 bis 25 Atmosphären liegt.

6. Prozess nach einem oder mehreren der Ansprüche 1-5, wobei der kombinierte Zufuhrstrom zu jeder Reaktionsstufe durch eine Einlasstemperatur (Tᵢₙ) gekennzeichnet ist und wobei Tᵢₙ zwischen 200 °C und 450 °C, vorzugsweise zwischen 250 °C und 350 °C liegt.

7. Prozess nach einem oder mehreren der Ansprüche 1-6, wobei der Abflussstrom aus jeder Reaktionsstufe durch eine Auslasstemperatur (Tₒᵤₜ) gekennzeichnet ist und wobei Tₒᵤₜ - Tᵢₙ ≥ 50°C in einer gegebenen Reaktionsstufe ist.

8. Prozess nach einem oder mehreren der Ansprüche 1-7, wobei in jede der Reaktionsstufen eine Sauerstoffumwandlung von ≥ 80% erreicht.

9. Prozess nach einem oder mehreren der Ansprüche 1-8, wobei das Katalysatorbett einen Moₐ V_{b} Nb_{c} X_{d} Katalysator umfasst, wobei X = mindestens eines von Folgendem ist: Te, Li, Sc, Na, Be, Mg, Ca, Sr, Ba, Ti, Zr, Hf, Y, Ta, Cr, Fe, Co, Ni, Ce, La, Zn, Cd, Hg, Al, Pb, As, Bi, Sb, U, Mn und W.

10. Prozess nach einem oder mehreren der Ansprüche 1-9, wobei die Kombination aus primärem Zufuhrstrom, sekundärem Zufuhrstrom und beliebigen zusätzlichen den Reaktorstufen bereitgestellten Strömen mindestens 90 Volumen-% des C2 - C6 Alkans umfasst.

11. Prozess nach einem oder mehreren der Ansprüche 1-9, wobei das C2 - C6 Alkan Ethan ist und der alkenhaltige Produktstrom Ethylen umfasst.

12. Prozess nach einem oder mehreren der Ansprüche 1-10, wobei der primäre Zufuhrstrom mindestens 90 Vol.-% an Ethan umfasst.

13. Prozess nach einem oder mehreren der Ansprüche 1-12, wobei die Gesamtumwandlung von Ethan aller Reaktionsstufen 10 % bis 50%, vorzugsweise 20 % bis 40% beträgt.

14. Prozess nach einem oder mehreren der Ansprüche 1-13, wobei die Gesamtselektivität von Ethylen in allen Reaktionsstufen ≥ 90% beträgt.

15. Prozess nach einem oder mehreren der Ansprüche 1-14, wobei ein Reaktorsystem frei von Zusetzen von Dampf oder inerten Verbindungen zum Reaktorsystem ist.

## Revendications

1. Procédé de production d'un flux de produit contenant un alcène par déshydrogénation oxydative, comprenant :
a) la fourniture d'un flux d'alimentation primaire comprenant un alcane C2 - C6 à travers un système de réacteur comportant un total de « n » étages de réaction en série, chacun des étages de réaction contenant un lit de catalyseur, dans lequel « n » varie de 2 à 20,
b) la fourniture d'un flux d'alimentation secondaire comprenant de l'oxygène à chacun des étages de réaction,
c) la production d'un flux d'effluent comprenant de l'alcène, de l'alcane et de l'oxygène provenant de chacun des étages de réaction ;
d) le refroidissement du flux d'effluent provenant des étages de réaction 1 à « n-1 » pour générer un flux d'alimentation d'entrée qui est acheminé vers l'étage de réaction suivante ; et
e) la production du flux de produit contenant de l'alcène dans le « n » ^{ème} étage de réaction ; dans lequel :
chacun des étages de réaction est sensiblement adiabatique ; le flux d'alimentation primaire comprend >= 75 % en volume de l'alcane C2-C6 et
la combinaison du flux d'alimentation primaire, du flux d'alimentation secondaire et de tous les flux supplémentaires fournis aux étages de réacteur comprend ≥ 75 % en volume de l'alcane C2 - C6 ; et
le flux d'effluent de chaque étage de réaction comprend ≤ 0,5 % molaire d'oxygène.

2. Procédé selon la revendication 1, dans lequel le flux d'alimentation primaire ou le flux d'alimentation d'entrée est combiné avec le flux d'alimentation secondaire pour constituer un flux d'alimentation combiné à chaque étage de réaction, de sorte que la concentration d'oxygène dans le flux d'alimentation combiné à chaque étage de réaction soit ≤ 5,0 % molaire.

3. Procédé selon une quelconque ou plusieurs des revendications 1-2, dans lequel le flux d'effluent provenant de chaque étage de réaction comprend ≤ 0,3 % molaire d'oxygène.

4. Procédé selon une quelconque ou plusieurs des revendications 1-3, dans lequel « n » varie de 3 à 10, de préférence de 5 à 8.

5. Procédé selon une quelconque ou plusieurs des revendications 1-4, dans lequel chaque étage de réaction est à une pression de 1 à 40 atmosphères, de préférence de 10 à 25 atmosphères.

6. Procédé selon une quelconque ou plusieurs des revendications 1-5, dans lequel le flux d'alimentation combiné vers chaque étage de réaction est **caractérisé par** une température d'entrée (Tᵢₙ) et dans lequel Tᵢₙ est compris entre 200°C et 450 °C, de préférence entre 250 °C et 350 °C.

7. Procédé selon une quelconque ou plusieurs des revendications 1-6, dans lequel le flux d'effluent provenant de chaque étage de réaction est **caractérisé par** une température de sortie (Tₒᵤₜ) et dans lequel Tₒᵤₜ - Tᵢₙ ≥ 50 °C à un étage de réaction donné.

8. Procédé selon une quelconque ou plusieurs des revendications 1-7, dans lequel chaque étage de réaction atteint une conversion d'oxygène ≥ 80 %.

9. Procédé selon une quelconque ou plusieurs des revendications 1-8, dans lequel le lit de catalyseur comprend un catalyseur Moₐ V_{b} Nb_{c} X_{d}, dans lequel X = au moins un des éléments suivants : Te, Li, Sc, Na, Be, Mg, Ca, Sr, Ba, Ti, Zr, Hf, Y, Ta, Cr, Fe, Co, Ni, Ce, La, Zn, Cd, Hg, Al, Pb, As, Bi, Sb, U, Mn et W.

10. Procédé selon une quelconque ou plusieurs des revendications 1-9, dans lequel la combinaison du flux d'alimentation primaire, du flux d'alimentation secondaire et de tous les flux supplémentaires fournis aux étages de réacteur comprend au moins 90 % en volume de l'alcane C2 - C6.

11. Procédé selon une quelconque ou plusieurs des revendications 1-9, dans lequel l'alcane C2 - C6 est l'éthane et le flux de produit contenant de l'alcène comprend de l'éthylène.

12. Procédé selon une quelconque ou plusieurs des revendications 1-10, dans lequel le flux d'alimentation primaire comprend au moins 90 % en volume d'éthane.

13. Procédé selon une quelconque ou plusieurs des revendications 1-12, dans lequel la conversion globale de l'éthane de tous les étages de réaction est de 10 % à 50 %, de préférence de 20 % à 40 %.

14. Procédé selon une quelconque ou plusieurs des revendications 1-13, dans lequel la sélectivité globale de l'éthylène à tous les étages de réaction est ≥ 90 %.

15. Procédé selon une quelconque ou plusieurs des revendications 1-14, dans lequel le système de réacteur est exempt d'ajout de vapeur ou de composés inertes au système de réacteur.
